Europäisches Patentamt

European Patent Office

Office européen des brevets

(19)

(11) Veröffentlichungsnummer: **0 266 686 B1**

(12) **EUROPÄISCHE PATENTSCHRIFT**

(45) Veröffentlichungstag der Patentschrift: **01.07.92**

(51) Int. Cl.⁵: **G01N 33/573**, G01N 33/546

(21) Anmeldenummer: **87115947.1**

(22) Anmeldetag: **30.10.87**

(54) **Latex-Agglutinations-Verfahren zum Nachweis von Anti-Streptokokken-Desoxyribonuclease B.**

(30) Priorität: **03.11.86 DE 3637253**

(43) Veröffentlichungstag der Anmeldung:
**11.05.88 Patentblatt 88/19**

(45) Bekanntmachung des Hinweises auf die
Patenterteilung:
**01.07.92 Patentblatt 92/27**

(84) Benannte Vertragsstaaten:
**AT BE CH DE ES FR GB IT LI LU NL SE**

(56) Entgegenhaltungen:
**EP-A- 0 040 799
EP-A- 0 049 849
EP-A- 0 061 857
GB-A- 2 058 083**

**CHEMICAL ABSTRACTS, Band 69, Nr. 1, 01
Juli 1968, Columbus, OH (US); J.NELSON et
al., Seite 131, Nr. 1382n**

**CHEMICAL ABSTRACTS, Band 102, Nr. 17, 29
April 1985, Columbus, OH (US);
J.STUERZEBECHER et al., Seite 249, Nr.
145184x**

(73) Patentinhaber: **BEHRINGWERKE Aktiengesellschaft
Postfach 1140
W-3550 Marburg 1(DE)**

(72) Erfinder: **Toth, Tibor, Dr.
Gründeberg 1
W-3550 Marburg(DE)**

(74) Vertreter: **Fischer, Hans-Jürgen, Dr. et al
Hoechst AG Zentrale Patentabteilung Postfach 80 03 20
W-6230 Frankturt am Main 80(DE)**

## Beschreibung

Die Erfindung betrifft eine stabilisierte Lösung von Streptokokken-DNase B und ihre Verwendung in einem Agglutinationsverfahren zum Nachweis von Antikörpern gegen Streptokokken-DNase B.

Streptokokken-DNase ist ein extrazelluläres Stoffwechselprodukt von Streptokokken A nach Lancefield. Streptokokken der Gruppe A produzieren vier verschiedene Desoxyribonucleasen (Streptodornasen), die sich säulenchromatographisch, elektrophoretisch und serologisch voneinander unterscheiden lassen. Die Bezeichnung dieser Isoenzyme mit A, B, C und D hat keinen Bezug zu der Streptokokken-Klassifizierung nach Lancefield. Die Streptokokken-DNase B ist ein Antigen, das im Menschen die Bildung spezifischer Antikörper induziert. Die Kenntnis der Konzentration dieser Antikörper im menschlichen Blut ist von diagnostischer Bedeutung.

Gegenüber der Anti-Streptolysin O-Bestimmung bietet die Anti-DNase-Bestimmung (Bestimmung von Antikörpern gegen Streptokokken-DNase B) diagnostische Vorteile. Vor allem bei Hautinfektionen kommt eine Erhöhung des Anti-Streptolysin-O-Gehaltes selten vor, während ein kräftiger Anstieg des Anti-DNase B-Titers beobachtet wird (J. clin. Invest. (1970) 49, 1405). Beim akuten rheumatischen Fieber, bei Glomerulonephritis und Infektionen der oberen Luftwege ist die Erhöhung der Konzentration von Antikörpern gegen Streptokokken-DNase B ausgeprägt und anhaltend. Es besteht deshalb ein Bedürfnis für eine Möglichkeit zur schnellen diagnostischen Bestimmung von Antikörpern gegen Streptokokken-DNase B.

Eine einfache und schnelle immunologische Methode zur Bestimmung von Anti-DNase B ist bisher nicht bekannt.

Die bekannten enzymatischen Methoden sind zeitaufwendig, erfordern besondere Sorgfalt oder einen großen apparativen Aufwand.

Allgemein ist als ein einfaches und rasch durchführbares Verfahren zum Nachweis oder zur Bestimmung eines Antigens oder Antikörpers der Latex-Agglutinationstest bekannt. Eine Ausführungsform besteht darin, daß zu einer Dispersion von Latexteilchen, an die ein Antikörper gebunden ist, eine bestimmte Menge des entsprechenden Antigens und die Probe gegeben werden, in welcher die Menge desselben, in ihr enthaltenen Antikörpers bestimmt werden soll.

Durch die Bindung von DNase-Antikörpern an Partikel werden diese für eine Reaktion mit DNase "sensibilisiert" und würden mit DNase unter Agglutination der Partikel reagieren. Bringt man jedoch zu prüfendes menschliches Serum, das Antikörper gegen DNase enthält, mit den sensibilisierten Partikeln und DNase zusammen, so reagieren diese Antikörper mit der DNase und die Agglutination der Partikel bleibt aus, wenn die DNase-Menge so gewählt ist, daß sie nicht größer als die Anti-DNase-Menge in dem Serum ist.

Demnach wird für eine Bestimmung von Antikörpern gegen Streptokokken-DNase B eine Lösung dieses Enzyms gebraucht, worin das Enzym genügend stabil ist, um eine für praktische Zwecke ausreichende Gebrauchsdauer eines entsprechenden Testsystems zu gewährleisten. Dieses Enzym verliert nämlich in wässriger Lösung an Aktivität. Bei den bekannten Anti-DNase-B-Testmethoden ist die DNase B deswegen lyophilisiert, und eine durch Auflösen eines solchen Lyophilisats in einer wässrigen Lösung erhaltene Enzymlösung muß nach Angaben der Hersteller innerhalb von etwa 48 Stunden verbraucht werden.

Als Zusätze zur DNase-Enzympräparation wurden $MgCl_2$, $MgSO_4$, $CaCl_2$ und Rinderserumalbumin in Imidazol-HCl-Pufferlösung, pH 8, sowie Gelatine und Polyhydroxyverbindungen wie Dextran oder Zucker beschrieben.

Es wurde jetzt gefunden, daß eine stabile DNase B-Lösung mit voller immunologischer Aktivität hergestellt werden kann, indem ein Proteaseinhibitor, vorzugsweise ein Benzamidiniumsalz zugesetzt wird.

Gegenstand der Erfindung ist daher ein Latex-Agglutinations-Verfahren zum Nachweis von Antikörpern gegen Streptokokken-Desoxyribonuclease B, wobei sich gegen Streptokokken-Desoxyribonuclease B gerichtete Antikörper auf den Teilchen einer Dispersion befinden und diese Dispersion mit einer Streptokokken-DNase B-Lösung und mit der zu bestimmenden Probe zusammengebracht wird, dadurch gekennzeichnet, daß die Streptokokken-DNase-Lösung einen Proteaseninhibitor enthält und die Streptokokken-DNase gegebenenfalls vernetzt wurde.

Dieses Verfahren erlaubt eine einfache und schnelle Bestimmung von Antikörpern gegen Streptokokken-Desoxyribonuclease B in wässriger Lösung mit hoher Spezifität, wobei das Reagenz eine gegenüber dem Stand der Technik verbesserte Gebrauchsdauer aufweist.

Der Proteaseinhibitor ist vorzugsweise ein Benzamidiniumsalz, besonders bevorzugt das Hydrochlorid.

Gegenstand der Erfindung ist weiterhin ein Agglutinations-Reagenz, bestehend aus einer Suspension oder Dispersion von Teilchen, vorzugsweise einer Dispersion von Polystyrol-Latex-Partikeln, auf denen sich DNase B-Antikörper befinden, und einer stabilisierten wässrigen Lösung von gegebenenfalls vernetzter Streptokokken-DNase B.

Zur Herstellung solcher mit DNase-Antikörpern beladenen Partikel werden Tiere, vorzugsweise Kaninchen, mit Streptokokken-DNase B immunisiert und Antikörper gegen diese DNase gewonnen. Diese Antikörper gegen Streptokokken-DNase B werden an suspendierte Partikel (Latex, suspendierte Erythrozyten) gebunden, indem eine Lösung dieser DNase-Antikörper mit den Partikeln in Kontakt gebracht wird.

Für die Immunisierung geeignete Streptokokken-DNase B-Präparationen können nach an sich bekannten Verfahren hergestellt werden, indem beispielsweise fermentativ gewonnene DNase B mit Ammoniumsulfat gefällt, der Rückstand dialysiert und durch säulenchromatographische Methoden oder Elektrofokussierung gereinigt wird. Mit einer solchen gereinigten DNase B-Enzym-Präparation können dann in Säugetieren Antikörper gegen die DNase B gewonnen werden. Eventuell vorhandene Unspezifitäten können nach den bekannten Methoden (Immunabsorption) entfernt werden.

Aber auch nach bekannten Verfahren herstellbare monoklonale Antikörper sollten geeignet sein.

Als Partikel-Suspensionen sind besonders Polymerlatices vorzugsweise Polystyrol-Dispersionen geeignet.

Als Beispiele seien genannt: Latices aus Homopolymeren und Copolymeren des Styrols oder seinen Derivaten wie Methylstyrol, Ethylstyrol oder Chlorstyrol, oder Acrylsäure oder ihren Estern wie Methylacrylat oder Ethylacrylat, oder Methacrylsäure oder ihren Derivaten wie Ethylmethacrylat, Acrylnitril oder Acrylamid, von Dienen wie Butadien, Chloropren oder Isopren, des Vinylchlorids, Vinylidenchlorids oder Vinylacetats.

Von diesen werden die Latices der Homopolymeren oder Copolymeren von Styrol, Acrylsäure oder Methylmethacrylat vorteilhaft verwendet.

Bevorzugt werden Latices mit einer Teilchengröße von etwa 0,05 bis 1 $\mu$m, insbesondere Teilchen einer Größe von 0,1 bis 0,6 $\mu$m.

Auch stabilisierte Erythrozyten können als Träger verwendet werden.

Die Beladung der Partikel mit dem Antikörper kann auf folgende Weise durchgeführt werden: Aus einem den Antikörper enthaltenden Antiserum wird in üblicher Weise eine Gammaglobulinfraktion ausgefällt, die in einer Konzentration von 0,01 bis 4 g/100 ml in einem 0,005 bis 0,2 molaren Puffer mit einem pH-Wert von 7 bis 9 gelöst wird. Als Puffer wird zweckmäßig ein Glycin-NaCl-, Phosphat-, Imidazol- oder Borat-Puffer verwendet. Bevorzugt wird eine 0,15 mol/l Glycin-NaCl-Pufferlösung von pH 8,2. Eine Suspension von Latexteilchen mit einer Konzentration von 0,1 bis 10 g/100 ml wird der Gammaglobulin-Lösung zugesetzt und die Mischung 1 bis 20 Stunden bei Raumtemperatur oder 0,5 bis 16 Stunden bei 35 bis 56°C stehengelassen oder gerührt. Die Suspension wird danach zentrifugiert, um nicht gebundenes Protein zu entfernen.

Der Antikörper kann auch nach bekannten Methoden kovalent an das Partikelmaterial gebunden werden.

Die mit Antikörpern beladenen Latexpartikel werden in einer Pufferlösung, bevorzugt einer Glycin-Natriumchlorid- oder Imidazol-Pufferlösung, insbesondere einer 0,1 bis 0,3 molaren Glycin-NaCl-Pufferlösung, die bis 3 g/100 ml vorzugsweise 1 bis 2 g/100 ml Human- oder Rinderserumalbumin enthalten kann, suspendiert, so daß bevorzugt eine Konzentration von 0,6 bis 1,2 g Latex in 100 ml Suspension erhalten wird.

Eine für das erfindungsgemäße Reagenz geeignete stabilisierte Streptokokken-DNase-Lösung kann durch Zugabe von 0,1 - 2 g/100 ml Benzamidinium-Hydrochlorid, 0,1 - 1,5 g/100 ml MgSO$_4$, 0,5 - 2 g/100 ml Albumin und/oder 0 - 1 g/100 ml eines Gelatinederivates, das durch begrenzte Hydrolyse von Gelatine und Vernetzen der Bruchstücke mit einem bifunktionellen Reagenz erhalten werden kann, beispielsweise Polygeline, und gegebenenfalls 0,1 g/100 ml eines Konservierungsmittels wie Natriumazid zu einer wässrigen 1-100 mg/100 ml Streptokokken-DNase B enthaltenden Lösung erhalten werden.

Wenn die DNase-B zuvor mit einem bifunktionellen Reagenz, beispielsweise Glutardialdehyd vernetzt wird, weist die Lösung besonders vorteilhafte Stabilitätseigenschaften auf und die Agglutination der Latex-Teilchen führt im Test zu besser sichtbaren Agglutinationsmustern.

Die Konzentration des Enzyms wird so eingestellt, daß sie pro ml etwa 2500 E enzymatischer Aktivität oder 50 E pro Test beträgt. Die Streptokokken DNase B braucht nicht durch aufwendige physikalische Methoden gereinigt zu werden. Es genügt eine Ammonium- oder Natrium-Sulfat-Fällung und Dialyse des Enzyms.

Folgende Beispiele erläutern die Erfindung.

Beispiel 1

Beladung der Partikel mit Anti-DNase B:

3

45 ml einer 50 g/l enthaltenden gamma-Globulin-Fraktion aus Anti-Streptokokken-DNase B-Serum vom Kaninchen wurden mit 500 ml Polystyrol-Latex (100 g/l Feststoff) mit einer Teilchengröße von 0,2 - 0,3μm in 0,15 molarer Glycin-NaCl-Pufferlösung, pH 8,2 vermischt und 3 Stunden bei 56°C im Wasserbad inkubiert. Der Feststoff wurde isoliert durch dreimaliges Zentrifugieren bei etwa 14.600 ×g, Dekantieren und Resuspendieren in je 1500 - 1700 ml Glycin-NaCl-Pufferlösung und danach in 4500 ml einer Lösung von 10 g/l Rinderserumalbumin in Glycin-NaCl-Pufferlösung resuspendiert.

Herstellung einer Streptokokken-DNase B-Lösung:

500 mg Streptokokken-DNase B-Lösung wurden in 100 ml phosphatgepufferter Kochsalzlösung, pH 7,1 (PBS) gelöst und mit 1 g Benzamidiniumchlorid und 2 g MgSO$_4$ versetzt. 10 g Polygeline und 2 g Albumin wurden in 100 ml phosphatgepufferter Kochsalzlösung, pH 7,1 hinzugefügt und das Volumen mit PBS auf 1000 ml aufgefüllt. Der Lösung wurden danach 0,5 g Natriumazid zugesetzt.

Ausführung des Anti-DNase B-Nachweises in Serum:

1 Tropfen (50 μl) zu prüfendes Humanserum wurde auf ein Feld einer Testplatte gegeben und 25 μl stabilisierte Streptokokken-DNase B-Lösung und 25 μl Streptokokken-DNase B-Latex zugegeben. Nach Durchmischen mittels Rührstäbchen wurde die Testplatte rotierend bewegt und nach fünf Minuten auf Agglutination geprüft.

Die folgende Tabelle veranschaulicht die Zuverlässigkeit des Verfahrens:

| Serum-Nr. | Anti-DNase B Toluidinblau Methode (E/ml) | Latex-Test gemäß Erfindung |
|---|---|---|
| 1 | 50 | − |
| 2 | 300 | + |
| 3 | 600 | + |
| 4 | 600 | + |
| 5 | 150 | − |
| 6 | 150 | − |
| 7 | 1200 | + |
| 8 | 75 | − |
| 9 | 50 | − |
| 10 | 50 | − |

+ bedeutet keine Agglutination; − Agglutination

Stabilitätsprüfungen bis zu einem Jahr zeigten, daß die verwendete Streptokokken DNase B-Lösung mindestens innerhalb dieses Zeitraums stabil war.

Beispiel 2

Ein nach Beispiel 1 hergestellter Anti-DNase-Latex wurde mit einer Streptokokken-DNase B-Präparation im Test eingesetzt, welche folgendermaßen hergestellt worden war:
100 mg Enzym-Präparation wurden unter Zugabe von 2,5 g Magnesiumsulfat in 1000 ml isotonischer Kochsalzlösung gelöst, und mit 10 g Polygeline, 2 g Benzamidiniumhydrochlorid und 1 g Natriumazid versetzt. Die Aktivität der Lösung wurde durch Verdünnen auf etwa 2500 E/ml eingestellt.

4

Beispiel 3

20 ml einer 45 g/l enthaltenden gamma-Globulin-Fraktion aus Anti-Streptokokken-DNase B-Serum vom Kaninchen wurde mit 150 ml Polystyrol-Latex (100 g/l Feststoff) mit einer Teilchengröße von 0,4 μm in 0,15 molarer Glycin-NaCl-Pufferlösung, pH 8,2 vermischt und 16 Stunden bei 37°C im Wasserbad inkubiert. Der Feststoff wurde isoliert durch dreimaliges Zentrifugieren bei etwa 14.600 xg, Dekantieren und Resuspendieren in je 1500 ml Glycin-NaCl-Pufferlösung und danach in 1500 ml einer Lösung von 10 g/l Human-Albumin in Glycin-NaCl-Pufferlösung resuspendiert.

100 mg Streptokokken-DNase B wurde in 100 ml Phosphat-Pufferlösung, pH 7,5-8 gelöst und mit 0,5 ml einer 2 g/100 ml Glutardialdehydlösung versetzt.

Nach 5 Stunden Rühren bei 4°C wurde die Lösung mit 2,5 g Magnesiumsulfat, die in 200 ml destilliertem Wasser gelöst worden waren, 10 g Polygeline, 2 g Benzamidiniumchlorid und 1 g Natriumazid versetzt. Das Volumen der Lösung wurde auf etwa 1 l aufgefüllt, so daß per ml etwa 2500 E/ml DNase B Aktivität erhalten wurden.

Die so hergestellte Lösung wurde im Test eingesetzt. Dazu wurden 1 Teil unverdünntes Patientenserum mit 0,5 Teilen DNase B-Präparation versetzt (z. B. 100 μl Patientenserum + 50 μl DNase B-Präparation). Danach wurde die Serumprobe 5-10 Min. bei Raumtemperatur stehengelassen. 50 μl der Serum-/Enzympräparation wurden auf ein Feld einer Testplatte gegeben und mit 25 μl DNase B-Latex versetzt. Nach guter Durchmischung mittels Rührstäbchen wurde die Testplatte 5 Minuten rotierend bewegt.

Beispiel 4

Nach Beispiel 1 hergestelltes Latex-Reagenz wurde mit einer Streptokokken-DNase B-Präparation im Test eingesetzt, welche folgendermaßen hergestellt worden war:

1000 mg Enzym-Präparation wurden in 1000 ml Phosphat-Pufferlösung pH 7,5, die 2,5 g Magnesiumsulfat enthielt, gelöst und mit 1,5 ml einer Lösung von 2 g Glutardialdehyd in 100 ml Wasser versetzt. Nach 5 Stunden Rühren bei 4°C wurde die Lösung von Trübungen und Ausflockungen durch Filtration oder Zentrifugation befreit. Zu der Lösung wurden 10 g Polygeline und 2 g Benzamidiniumhydrochlorid gegeben und mit 1 g Natriumazid versetzt. Die Aktivität der Lösung wurde durch Vergleich mit einem Standard durch Verdünnen mit isotonischer Kochsalzlösung auf 2500 E/ml eingestellt. Die so hergestellte Lösung wurde im Test, wie unter 1 beschrieben, eingesetzt, wobei an einem größeren Kollektiv von Probandenseren (500 Seren) mit der Toluidinblau-Methode eine Übereinstimmung von mehr als 90 % erzielt wurde.

**Patentansprüche**

**Patentansprüche für folgende Vertragsstaaten : AT, BE, CH, DE, FR, GB, IT, LI, LU, NL, SE**

1. Latex-Agglutinations-Verfahren zum Nachweis von Antikörpern gegen Streptokokken-Desoxyribonuclease B, wobei sich gegen Streptokokken-Desoxyribonuclease B gerichtete Antikörper auf den Teilchen einer Suspension oder Dispersion befinden und eine solche Suspension oder Dispersion mit einer Streptokokken-DNase B-Lösung und mit der zu bestimmenden Probe zusammengebracht wird, dadurch gekennzeichnet, daß die Streptokokken-DNase-Lösung einen Proteaseninhibitor enthält und die Streptokokken-DNase gegebenenfalls vernetzt wurde.

2. Verfahren nach Anspruch 1, dadurch gekennzeichnet, daß der Proteaseinhibitor ein Benzamidiniumsalz ist.

3. Verfahren nach Anspruch 1, dadurch gekennzeichnet, daß die stabilisierte DNase B-Präparation 0,1-1 mg/ml DNase B, bis zu 2 g/100 ml einer abgebauten und vernetzten Gelatine, bis zu 3 g/100 ml Benzamidiniumchlorid und 0 - 5 g/100 ml eines Magnesiumsalzes enthält.

4. Verfahren nach Anspruch 1, dadurch gekennzeichnet, daß die stabilisierte Streptokokken-DNase B-Präparation bis zu 3 g/100 ml Aprotinin, bis zu 2 g/100 ml abgebaute und vernetzte Gelatine und 0 - 5 g/100 ml eines Magnesiumsalzes enthält.

5. Verfahren nach Anspruch 1, dadurch gekennzeichnet, daß die Streptokokken-DNase B-Präparation Streptokokken-DNase B enthält, die mit einem bifunktionellen Reagenz behandelt wurde.

6. Verfahren nach Anspruch 1, dadurch gekennzeichnet, daß das Latex ein Polystyrol-Latex ist.

**7.** Verfahren nach Anspruch 1, dadurch gekennzeichnet, daß die Antikörper solche aus einem Säugetier sind.

**8.** Verfahren nach Anspruch 1, dadurch gekennzeichnet, daß die Antikörper vom Kaninchen sind.

**9.** Agglutinations-Reagenz, bestehend aus einer Suspension oder Dispersion von Teilchen, auf denen sich DNase B-Antikörper befinden, und einer wässrigen Lösung von Streptokokken-DNase B, die einen Proteaseinhibitor enthält.

**10.** Reagenz nach Anspruch 9, dadurch gekennzeichnet, daß die Teilchen aus Polystyrol-Latex bestehen.

**Patentansprüche für folgenden Vertragsstaat : ES**

**1.** Latex-Agglutinations-Verfahren zum Nachweis von Antikörpern gegen Streptokokken-Desoxyribonuclease B, wobei sich gegen Streptokokken-Desoxyribonuclease B gerichtete Antikörper auf den Teilchen einer Suspension oder Dispersion befinden und eine solche Suspension oder Dispersion mit einer Streptokokken-DNase B-Lösung und mit der zu bestimmenden Probe zusammengebracht wird, dadurch gekennzeichnet, daß die Streptokokken-DNase-Lösung einen Proteaseninhibitor enthält und die Streptokokken-DNase gegebenenfalls vernetzt wurde.

**2.** Verfahren nach Anspruch 1, dadurch gekennzeichnet, daß der Proteaseinhibitor ein Benzamidiniumsalz ist.

**3.** Verfahren nach Anspruch 1, dadurch gekennzeichnet, daß die stabilisierte DNase B-Präparation 0,1-1 mg/ml DNase B, bis zu 2 g/100 ml einer abgebauten und vernetzten Gelatine, bis zu 3 g/100 ml Benzamidiniumchlorid und 0 - 5 g/100 ml eines Magnesiumsalzes enthält.

**4.** Verfahren nach Anspruch 1, dadurch gekennzeichnet, daß die stabilisierte Streptokokken-DNase B-Präparation bis zu 3 g/100 ml Aprotinin, bis zu 2 g/100 ml abgebaute und vernetzte Gelatine und 0 - 5 g/100 ml eines Magnesiumsalzes enthält.

**5.** Verfahren nach Anspruch 1, dadurch gekennzeichnet, daß die Streptokokken-DNase B-Präparation Streptokokken-DNase B enthält, die mit einem bifunktionellen Reagenz behandelt wurde.

**6.** Verfahren nach Anspruch 1, dadurch gekennzeichnet, daß das Latex ein Polystyrol-Latex ist.

**7.** Verfahren nach Anspruch 1, dadurch gekennzeichnet, daß die Antikörper solche aus einem Säugetier sind.

**8.** Verfahren nach Anspruch 1, dadurch gekennzeichnet, daß die Antikörper vom Kaninchen sind.

**Claims**
**Claims for the following Contracting States : AT, BE, CH, DE, FR, GB, IT, LI, LU, NL, SE**

**1.** A latex agglutination method for the detection of antibodies against streptococcal deoxyribonuclease B, in which antibodies directed against streptococcal deoxyribonuclease B are located on the particles of a suspension or dispersion, and a suspension or dispersion of this type is mixed with a streptococcal DNase B solution and with the sample which is to be determined, which comprises the streptococcal DNase solution containing a protease inhibitor, and the streptococcal DNase having been, where appropriate, crosslinked.

**2.** The method as claimed in claim 1, wherein the protease inhibitor is a benzamidinium salt.

**3.** The method as claimed in claim 1, wherein the stabilized DNase B preparation contains 0.1-1 mg/ml DNase B, up to 2 g/100 ml of a degraded and crosslinked gelatin, up to 3 g/100 ml benzamidinium chloride, and 0 - 5 g/100 ml of a magnesium salt.

**4.** The method as claimed in claim 1, wherein the stabilized streptococcal DNase preparation contains up to 3 g/100 ml aprotinin, up to 2 g/100 ml degraded and crosslinked gelatin, and 0 - 5 g/100 ml of a magnesium salt.

5. The method as claimed in claim 1, wherein the streptococcal DNase B preparation contains streptococcal DNase B which has been treated with a bifunctional reagent.

6. The method as claimed in claim 1, wherein the latex is a polystyrene latex.

7. The method as claimed in claim 1, wherein the antibodies are those from a mammal.

8. The method as claimed in claim 1, wherein the antibodies are from a rabbit.

9. An agglutination reagent composed of a suspension or dispersion of particles on which are located DNase B antibodies, and of an aqueous solution of streptococcal DNase B which contains a protease inhibitor.

10. A reagent as claimed in claim 9, wherein the particles are composed of polystyrene latex.

**Claims for the following Contracting State : ES**

1. A latex agglutination method for the detection of antibodies against streptococcal deoxyribonuclease B, in which antibodies directed against streptococcal deoxyribonuclease B are located on the particles of a suspension or dispersion, and a suspension or dispersion of this type is mixed with a streptococcal DNase B solution and with the sample which is to be determined, which comprises the streptococcal DNase solution containing a protease inhibitor, and the streptococcal DNase having been, where appropriate, crosslinked.

2. The method as claimed in claim 1, wherein the protease inhibitor is a benzamidinium salt.

3. The method as claimed in claim 1, wherein the stabilized DNase B preparation contains 0.1-1 mg/ml DNase B, up to 2 g/100 ml of a degraded and crosslinked gelatin, up to 3 g/100 ml benzamidinium chloride, and 0 - 5 g/100 ml of a magnesium salt.

4. The method as claimed in claim 1, wherein the stabilized streptococcal DNase B preparation contains up to 3 g/100 ml aprotinin, up to 2 g/100 ml degraded and crosslinked gelatin, and 0 - 5g/100ml of a magnesium salt.

5. The method as claimed in claim 1, wherein the streptococcal DNase B preparation contains streptococcal DNase B which has been treated with a bifunctional reagent.

6. The method as claimed in claim 1, wherein the latex is a polystyrene latex.

7. The method as claimed in claim 1, wherein the antibodies are those from a mammal.

8. The method as claimed in claim 1, wherein the antibodies are from a rabbit.

**Revendications**
**Revendications pour les Etats contractants suivants : AT, BE, CH, DE, FR, GB, IT, LI, LU, NL, SE**

1. Procédé d'agglutination de latex pour l'identification des anticorps contre la désoxyribonucléase B des streptocoques, dans lequel les anticorps dirigés contre la désoxyribonucléase B des streptocoques se trouvent sur les particules d'une suspension ou d'une dispersion et cette suspension ou dispersion est mise au contact d'une solution de DNase B de streptocoque et de l'échantillon à doser, caractérisé en ce que la solution de DNase de streptocoque contient un inhibiteur de protéase et la DNase de streptocoque a été éventuellement réticulée.

2. Procédé selon la revendication 1, caractérisé en ce que l'inhibiteur de protéase est un sel de benzamidinium.

EP 0 266 686 B1

**3.** Procédé selon la revendication 1, caractérisé en ce que la préparation de DNase B stabilisée contient de 0,1 à 1 mg/ml de DNase B, jusqu'à 2 g/100 ml d'une gélatine dégradée et réticulée, jusqu'à 3 g/100 ml de chlorure de benzamidinium et de 0 à 5 g/100 ml d'un sel de magnésium.

**4.** Procédé selon la revendication 1, caractérisé en ce que la préparation de DNase B de streptocoque stabilisée contient jusqu'à 3 g/100 ml d'aprotinine, jusqu'a 2 g/100 ml de gélatine dégradée et réticulée et de 0 à 5 g/100 ml d'un sel de magnésium.

**5.** Procédé selon la revendication 1, caractérisé en ce que la préparation de DNase B de streptocoque contient de la DNase B de streptocoque qui a été traitée avec un réactif bifonctionnel.

**6.** Procédé selon la revendication 1, caractérisé en ce que le latex est un latex de polystyrène.

**7.** Procédé selon la revendication 1, caractérisé en ce que les anticorps sont des anticorps de mammifère.

**8.** Procédé selon la revendication 1, caractérisé en ce que les anticorps sont des anticorps de lapin.

**9.** Réactif d'agglutination, comprenant une suspension ou une dispersion de particules, sur lesquelles se trouvent les anticorps de DNase B, et une solution aqueuse de DNase B de streptocoque qui contient un inhibiteur de protéase.

**10.** Réactif selon la revendication 9, caractérisé en ce que les particules se composent de latex de polystyrène.

**Revendications pour l'Etat contractant suivant : ES**

**1.** Procédé d'agglutination de latex pour l'identification des anticorps contre la désoxyribonucléase B des streptocoques, dans lequel les anticorps dirigés contre la désoxyribonucléase B des streptocoques se trouvent sur les particules d'une suspension ou d'une dispersion et cette suspension ou dispersion est mise au contact d'une solution de DNase B de streptocoque et de l'échantillon à doser, caractérisé en ce que la solution de DNase de streptocoque contient un inhibiteur de protéase et la DNase de streptocoque a été éventuellement réticulée.

**2.** Procédé selon la revendication 1, caractérisé en ce que l'inhibiteur de protéase est un sel de benzamidinium.

**3.** Procédé selon la revendication 1, caractérisé en ce que la préparation de DNase B stabilisée contient de 0,1 à 1 mg/ml de DNase B, jusqu'à 2 g/100 ml d'une gélatine dégradée et réticulée, jusqu'à 3 g/100 ml de chlorure de benzamidinium et de 0 à 5 g/100 ml d'un sel de magnésium.

**4.** Procédé selon la revendication 1, caractérisé en ce que la préparation de DNase B de streptocoque stabilisée contient jusqu'à 3 g/100 ml d'aprotinine, jusqu'à 2 g/100 ml de gélatine dégradée et réticulée et de 0 à 5 g/100 ml d'un sel de magnésium.

**5.** Procédé selon la revendication 1, caractérisé en ce que la préparation de DNase B de streptocoque contient de la DNase B de streptocoque qui a été traitée avec un réactif bifonctionnel.

**6.** Procédé selon la revendication 1, caractérisé en ce que le latex est un latex de polystyrène.

**7.** Procédé selon la revendication 1, caractérisé en ce que les anticorps sont dos anticorps de mammifère.

**8.** Procédé selon la revendication 1, caractérisé en ce que les anticorps sont des anticorps de lapin.

8